# EUROPÄISCHE PATENTANMELDUNG

(11) **EP 2 039 678 A1**
(43) Veröffentlichungstag der Anmeldung: **25.03.2009**
(21) Anmeldenummer: 07116638.3
(22) Anmeldetag: 18.09.2007
(51) Int. Cl.: C07C 227/08, C07C 229/30, C07D 307/58

(54) **Verfahren zum Herstellen von 4-Aminobut-2-enoliden**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur Herstellung von 4-Amino-but-2-enoliden sowie von entsprechenden Intermediaten bzw. Ausgangsverbindungen, welche in dem erfindungsgemäßen Verfahren durchlaufen bzw. verwendet werden.

## Beschreibung

Die vorliegende Erfindung betrifft ein Verfahren zur Herstellung von 4-Amino-but-2-enoliden sowie von entsprechenden Intermediaten bzw. Ausgangsverbindungen, welche in dem erfindungsgemäßen Verfahren durchlaufen bzw. verwendet werden. Weiterer Gegenstand der vorliegenden Erfindung sind Verfahren zur Herstellung der entsprechenden Intermediate bzw. Ausgangsverbindungen.

Bestimmte substituierte 4-Aminobut-2-enolid-Verbindungen sind als insektizid wirksame Verbindungen aus der EP 0 539 588 A1 bekannt. Darüber hinaus beschreiben auch die prioritätsälteren, jedoch nicht-vorveröffentlichten Patentanmeldungen PCT/EP2007/002386, PCT/EP2007/0023385 und PCT/EP2007/002392 entsprechende insektizid wirksame 4-Aminobut-2-enolid-Verbindungen.

Im Allgemeinen werden Enaminocarbonylverbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

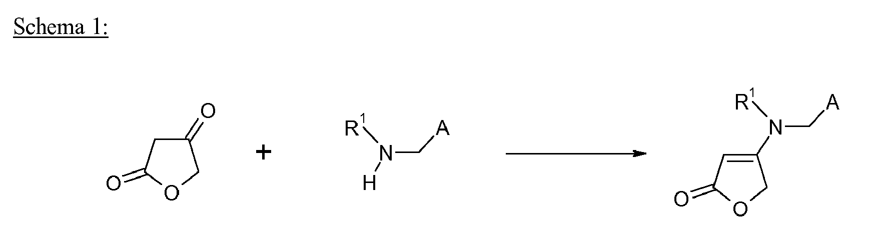

Nachteilig an diesem Verfahren ist insbesondere, dass man wasserfreie Tetronsäure als Ausgangsverbindung benötigt, deren Herstellung aufwendig und kostenintensiv ist.

So wird Tetronsäure im Allgemeinen ausgehend von Acetessigester über eine Bromierung und anschließende Hydrierung hergestellt (vgl. Synthetic Communication, 11(5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben. Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt. Der Nachteil an diesem Verfahren besteht darin, dass die Isolierung der Tetronsäure nur durch Hochvakuumdestillation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestem ausgegangen wird. Dieses ebenfalls mehrstufige und aufwändige Verfahren liefert die gewünschte Verbindung ebenfalls nur mit einer mäßigen Gesamtausbeute von 65 %.

In Tetrahedron Letters, Nr. 31, Seiten 2683 und 2684 (1974) ist die Herstellung von Tetronsäure und einer entsprechenden Enaminocarbonylverbindung beschrieben. Die dort beschriebene Synthese ist in folgendem Schema 2 wiedergegeben. Als Edukt wird dabei Acetylendicarbonsäuredimethylester eingesetzt.

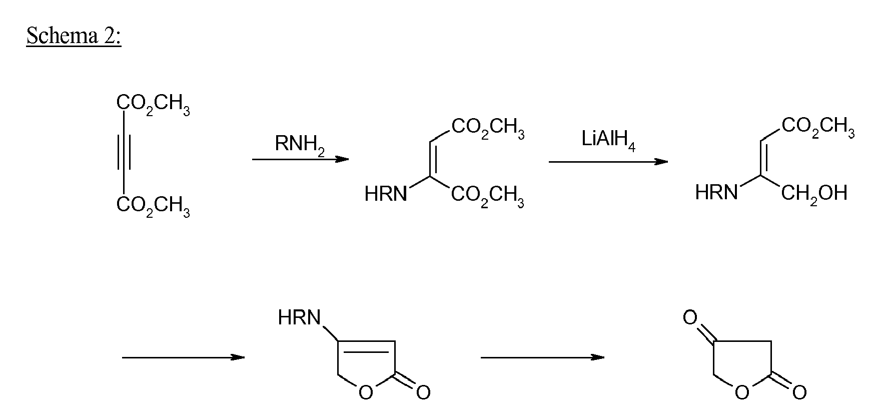

Nachteilig an diesem Verfahren sind die geringe Gesamtausbeute von nur 30 % sowie das Erfordernis, kostenintensive Edukte, beispielsweise Lithiumaluminiumhydrid (LiAlH4), als Reagenzien verwenden zu müssen.

Ferner ist aus dem Stand der Technik ein Verfahren zur Herstellung von 4-Aminobut-2-enoliden ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40%.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %.

In der vorstehend erwähnten, prioritätsälteren, nicht vorveröffentlichten Internationalen Patentanmeldung PCT/EP2007/002386 wird die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en, beschrieben (vgl. Herstellungsbeispiel, Verfahren 2, Beispiel (3)). Die PCT/EP2007/002386 beschreibt auch die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben. Im erfindungsgemäßen Verfahren werden dagegen preiswerte und sicherheitstechnologisch einfacher handhabbare Basen, z.B. NaOH verwendet.

Die Aufgabe der vorliegenden Erfindung bestand in der Bereitstellung eines neuen, wirtschaftlichen Verfahrens zum Herstellen von 4-Aminobut-2-enolidverbindungen und zum Herstellen von Ausgangsverbindungen für dieses Verfahren.

Gegenstand der vorliegenden Erfindung ist daher ein Verfahren zum Herstellen von Verbindungen der allgemeinen Formel (IVa) oder (IVb) 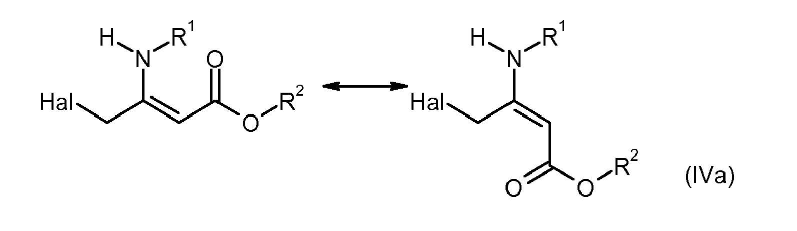 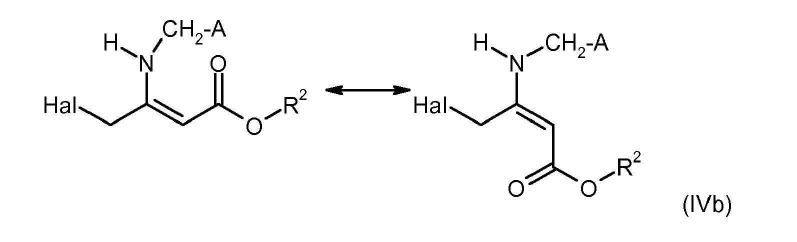 in welcher
- A: für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
- A: für einen Rest Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist,
oder
- A: für einen Rest 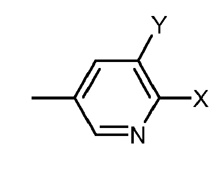 steht,
in welchem
- X: für Halogen, Alkyl oder Halogenalkyl steht
- Y: für Halogen, Alkyl, Halogenalkyl, Halogenalkoxy, Azido oder Cyan steht,
- R¹: für Alkyl, Halogenalkyl, Alkenyl, Halogenalkenyl, Alkinyl, Cycloalkyl, Cycloalkylalkyl, Halogencycloalkyl, Alkoxy, Alkoxyalkyl, Halogencycloalkylalkyl oder Arylalkyl steht,
- R²: für C₁-C₁₂-Alkyl, C₅-C₈-Aryl oder Arylalkyl, bevorzugt für C₁-C₆ Alkyl, besonders bevorzugt für Methyl oder Ethyl steht und
- Hal: für Chlor, Brom oder Jod steht,
dadurch gekennzeichnet, dass
4-Halogenacetessigester der allgemeinen Formel (II) 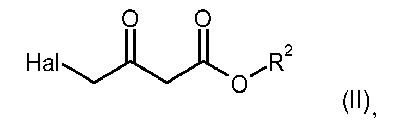 in welcher R² und Hal die oben genannte Bedeutung haben,
a) mit einem Amin der Formel (IIIa) 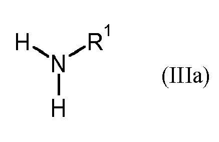 in welcher R¹ die oben genannte Bedeutung hat,
   zur Verbindung der Formel (IVa) umgesetzt wird,
   oder
b) mit einem Amin der Formel (IIIb)

   H₂N-CH₂-A (IIIb)

   in welcher A die oben genannte Bedeutung hat,
   zur Verbindung der Formel (IVb) umgesetzt wird.
   Ein weiterer Gegenstand der vorliegenden Erfindung ist ein Verfahren zum Herstellen von 4-Aminobut-2-enoliden der allgemeinen Formel (I) 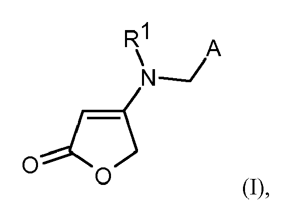 in welcher
   A und R¹ die oben genannten Bedeutungen haben,
   dadurch gekennzeichnet, dass 4-Halogenacetessigester der allgemeinen Formel (II) 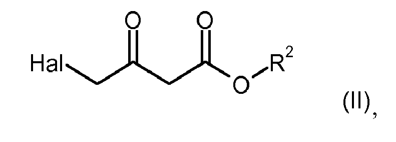 in welcher R² und Hal die oben genannte Bedeutung haben, mit

a) einem Amin der Formel (IIIa) 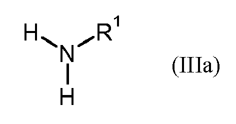 in welcher R¹ die oben genannte Bedeutung hat,
   zu einer Verbindung der Formel (IVa) 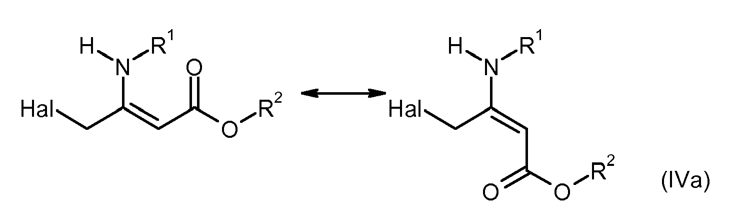 umgesetzt wird, anschließend die Verbindung der Formel (IVa) in Gegenwart eines Lösungsmittels thermisch zu einer Verbindung der Formel (Va) cyclisiert wird 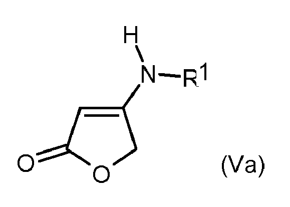 und die Verbindung der Formel (Va) im letzten Schritt mit einer Verbindung der Formel (VIa)

   A-CH₂-E (VIa)

   wobei A die oben genannte Bedeutung hat und E für eine Abgangsgruppe steht, zur Verbindung der Formel (I) umgesetzt wird; oder
b) mit einem Amin der Formel (IIIb)

   H₂N-CH₂-A (IIIb)

   in welcher A die oben genannte Bedeutung hat,
   zu einer Verbindung der Formel (IVb) 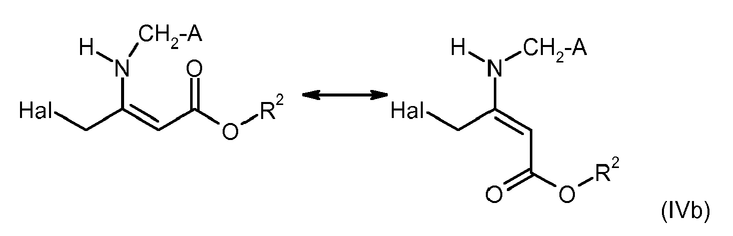 umgesetzt wird, anschließend die Verbindung der Formel (IVb) in Gegenwart eines Lösungsmittels thermisch zu einer Verbindung der Formel (Vb) cyclisiert wird 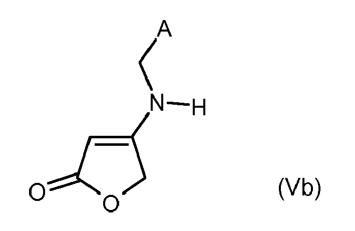 und die Verbindung der Formel (Vb) im letzten Schritt mit einer Verbindung der Formel (VIb)

   R¹-E (VIb)

   wobei R¹ die oben genannte Bedeutung hat und E für eine Abgangsgruppe steht, zur Verbindung der Formel (I) umgesetzt wird.

Mögliche Abgangsgruppen E sind Halogen, wie Chlor, Brom oder Jod oder aktivierte Hydroxyverbindungen wie Mesylat, Tosylat oder SO₂Me.

Überraschenderweise lassen sich die 4-Aminobut-2-enolide der Formel (I) unter den erfindungsgemäßen Bedingungen mit sehr guten Ausbeuten in hoher Reinheit herstellen, womit das erfindungsgemäße Verfahren die oben genannten Nachteile überwindet. Gegenüber dem aus dem Stand der Technik bekannten Verfahren, das von 4-Chloracetessigester ausgeht und diesen mit Aminen umsetzt (Heterocycles, Vol. 27, No. 8, 1988, 1907 - 1923) konnten die Ausbeuten durch das erfindungsgemäße Verfahren verdoppelt werden. Auch gegenüber dem in der PCT/EP2007/002386 beschriebenen Verfahren zur Herstellung von 4-Aminobut-2-enoliden (vgl. oben) konnte die Ausbeute durch das erfindungsgemäße Verfahren wesentlich erhöht werden.

Ein weiterer Gegenstand der vorliegenden Erfindung sind Verbindungen der Formel (IVa) oder (IVb) 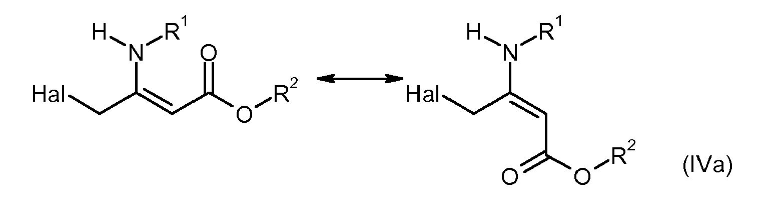 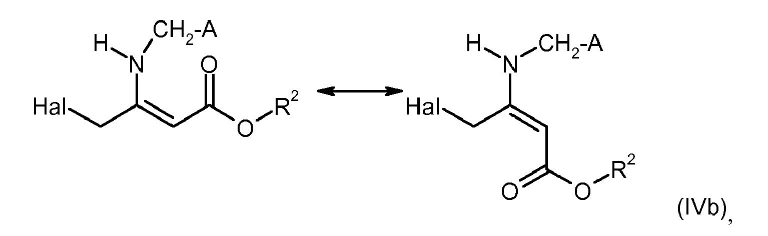 in welcher
- Hal: für Chlor steht und
- R¹, A und R²: wie oben definiert sind.

Die Produkte des erfindungsgemäßen Verfahrens sind durch die Formel (I) allgemein definiert. Bevorzugte, besonders bevorzugte und ganz besonders bevorzugte Substituenten bzw. Bereiche der in der oben erwähnten Formel (I) aufgeführten Reste werden im Folgenden erläutert.
- A: steht bevorzugt für 6-Fluor-pyrid-3-yl, 6-Chlor-pyrid-3-yl, 6-Brom-pyrid-3-yl, 6-Methyl- pyrid-3-yl, 6-Trifluormethyl-pyrid-3-yl, 6-Trifluormethoxypyrid-3-yl, 6-Chlor-1,4- pyridazin-3-yl, 6-Methyl-1,4-pyridazin-3-yl, 2-Chlor-1,3-thiazol-5-yl oder 2-Methyl-1,3- thiazol-5-yl, 2-Chlor-pyrimidin-5-yl, 2-Trifluormethyl-pyrimidin-5-yl, 5,6-Difluor-pyrid-3- yl, 5-Chlor-6-fluor-pyrid-3-yl, 5-Brom-6-fluor-pyrid-3-yl, 5-Iod-6-fluor-pyrid-3-yl, 5-Fluor- 6-chlor-pyrid-3-yl, 5,6-Dichlor-pyrid-3-yl, 5-Brom-6-chlor-pyrid-3-yl, 5-Iod-6-chlor-pyrid- 3-yl, 5-Fluor-6-brom-pyrid-3-yl, 5-Chlor-6-brom-pyrid-3-yl, 5,6-Dibrom-pyrid-3-yl, 5- Fluor-6-iod-pyrid-3-yl, 5-Chlor-6-iod-pyrid-3-yl, 5-Brom-6-iod-pyrid-3-yl, 5-Methyl-6- fluor-pyrid-3-yl, 5-Methyl-6-chlor-pyrid-3-yl, 5-Methyl-6-brom-pyrid-3-yl, 5-Methyl-6-iod- pyrid-3-yl, 5-Difluormethyl-6-fluor-pyrid-3-yl, 5-Difluormethyl-6-chlor-pyrid-3-yl, 5- Difluormethyl-6-brom-pyrid-3-yl oder 5-Difluormethyl-6-iod-pyrid-3-yl.
- R¹: steht bevorzugt für gegebenenfalls durch Fluor substituiertes C₁-C₅-Alkyl, C₂-C₅-Alkenyl, C₃-C₅-Cycloalkyl, C₃-C₅-Cycloalkylalkyl oder Alkoxyalkyl.
- A: steht besonders bevorzugt für den Rest 6-Fluor-pyrid-3-yl, 6-Chlor-pyrid-3-yl, 6- Brompyrid-3-yl, 6-Chlor-1,4-pyridazin-3-yl, 2-Chlor-1,3-thiazol-5-yl, 2-Chlor-pyrimidin-5- yl, 5-Fluor-6-chlor-pyrid-3-yl, 5,6-Dichlor-pyrid-3-yl, 5-Brom-6-chlor-pyrid-3-yl, 5-Fluor- 6-brom-pyrid-3-yl, 5-Chlor-6-brom-pyrid-3-yl, 5,6-Dibrom-pyrid-3-yl, 5-Methyl-6-chlor- pyrid-3-yl, 5-Chlor-6-iod-pyrid-3-yl oder 5-Difluormethyl-6-chlor-pyrid-3-yl.
- R¹: steht besonders bevorzugt für Methyl, Ethyl, Propyl, Vinyl, Allyl, Propargyl, Cyclopropyl, Alkoxyalkyl, 2-Fluor-ethyl, 2,2-Difluor-ethyl oder 2-Fluor-cyclopropyl.
- A: steht ganz besonders bevorzugt für den Rest 6-Fluor-pyrid-3-yl, 6-Chlor-pyrid-3-yl, 6- Brom-pyrid-3-yl, 2-Chlor-1,3-thiazol-5-yl, 5-Fluor-6-chlor-pyrid-3-yl, 5-Fluor-6-brom- pyrid-3-yl oder 5,6-Dichlor-pyrid-3-yl.
- R¹: steht ganz besonders bevorzugt für Methyl, Ethyl, n-Propyl, n-Prop-2-enyl, n-Prop-2-inyl, Cyclopropyl, Methoxyethyl, 2-Fluorethyl, oder 2,2-Difluor-ethyl.

Das erfindungsgemäße Verfahren kann anhand der folgenden Schemata 3a und 3b erläutert werden:

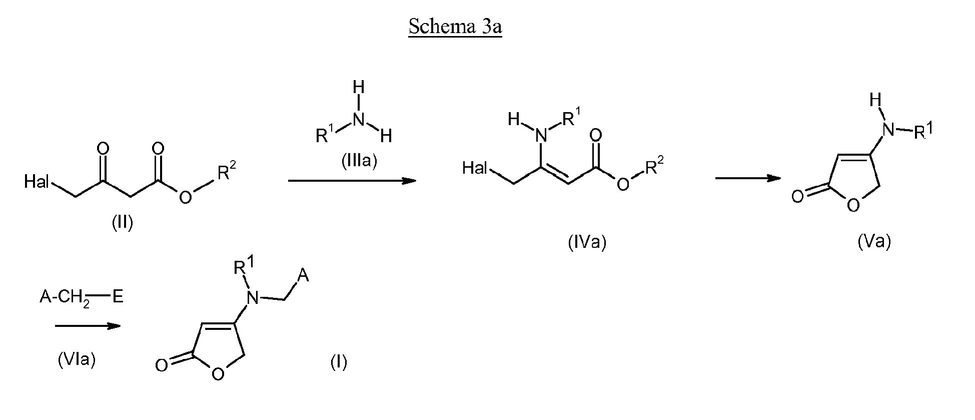

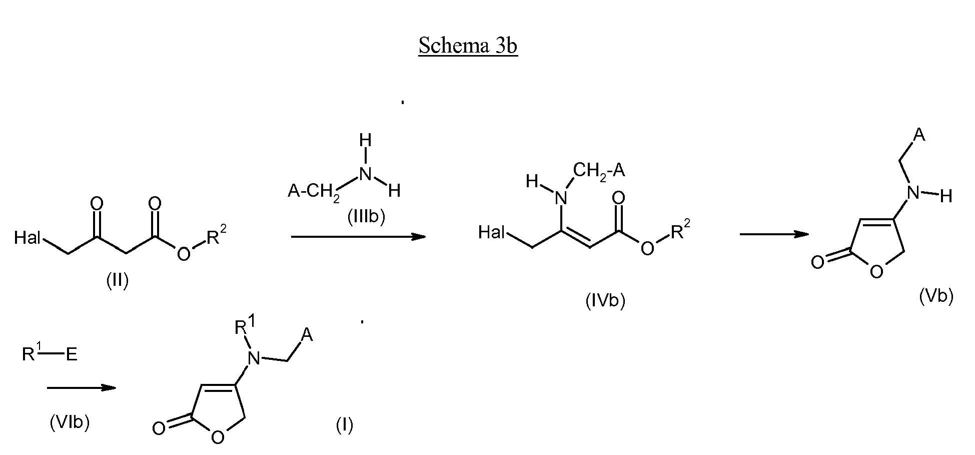

Im Rahmen der vorliegenden Erfindung ist "Alkyl" definiert als lineares oder verzweigtes C₁₋₁₂ Alkyl wie Methyl, Ethyl, n- oder iso-Propyl, n-, iso-, sec- oder tert-Butyl, n-Pentyl, n-Hexyl, n-Heptyl, n-Octyl, n-Nonyl, n-Decyl, n-Undecyl, n-Dodecyl und weitere, bevorzugt ist C₁₋₆ Alkyl, besonders bevorzugt ist C₁₋₄ Alkyl.

"Alkenyl" ist definiert als lineares oder verzweigtes C₂₋₁₂ Alkenyl, das mindestens eine Doppelbindung aufweist, wie Vinyl, Allyl, 1-Propenyl, Isopropenyl, 1-Butenyl, 2-Butenyl, 3-Butenyl, 1,3-Butandienyl, 1-Pentenyl, 2-Pentenyl, 3-Pentenyl, 4-Pentenyl, 1,3 Pentandienyl, 1-Hexenyl, 2-Hexenyl, 3-Hexenyl, 4-Hexenyl, 5-Hexenyl, 1,4-Hexandienyl und weitere, bevorzugt ist C₂₋₆ Alkenyl, besonders bevorzugt ist C₂₋₄ Alkenyl.

"Alkinyl" ist definiert als C₃₋₁₂ Alkinyl, das mindestens eine Dreifachbindung und optional zusätzlich eine oder mehrere Doppelbindungen aufweist, wie Ethinyl, 1-Propinyl, Propargyl, bevorzugt ist C₃₋₆ Alkinyl, besonders bevorzugt ist C₃₋₄ Alkinyl.

Jeder Alkyl-Bestandteil in den Resten "Alkoxy", "Alkoxyalkyl", "Haloalkyl", "Cycloalkylalkyl", "Halogencycloalkylalkyl", "Arylalkyl" und ähnlichen Resten ist wie oben für "Alkyl" beschrieben definiert. Das Gleiche gilt für Reste, die einen Alkenyl- oder Alkinyl-Bestandteil enthalten.

"Cycloalkyl" ist definiert als C₃₋₈ Cycloalkyl wie Cyclopropyl, Cyclobutyl, Cyclopentyl, Cyclohexyl, Cycloheptyl, Cyclooctyl und Ähnliche, bevorzugt ist C₃₋₆ Cycloalkyl.

"Aryl" ist definiert als aromatischer Rest mit 6 bis 14 Kohlenstoffatomen, bevorzugt ist Phenyl.

"Arylalkyl" ist definiert als beispielsweise Benzyl, Phenylethyl oder α-Methylbenzyl, bevorzugt ist Benzyl.

"Halogen" ist definiert als Fluor, Chlor, Brom oder Iod, bevorzugt sind Fluor oder Chlor.

Jeder Halogen-Bestandteil in den Resten "Halogenalkyl", "Halogenalkenyl", "Halogenalkinyl", "Halogencycloalkyl", "Halogencycloalkylalkyl" und ähnlichen Resten ist wie oben für Halogen beschrieben definiert.

Die gemäß der vorliegenden Erfindung verwendeten 4-Halogenacetessigester sind Verbindungen gemäß der allgemeinen Formel (II) 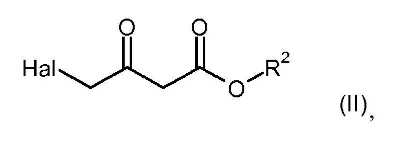 in welcher R² für C₁-C₁₂-Alkyl, C₅-C₈-Aryl oder Alkylaryl, steht, bevorzugt für C₁ - C₆ Alkyl, besonders bevorzugt für Methyl oder Ethyl, und Hal für C₁, Br oder I steht, bevorzugt für C₁ oder Br, besonders bevorzugt für C₁.

Die 4-Halogenacetessigsäureesterverbindungen sind kommerziell erhältlich oder können nach literaturbekannten Verfahren leicht hergestellt werden (Organic Syntheses (1973), 53, 1882; US Pat. No 4,468,356).

Die Umsetzung des 4-Halogenacetessigesters mit dem Amin der Formel (IIIa) oder (IIIb) zur Verbindung mit der Formel (IVa) bzw. (IVb) kann in Gegenwart eines Lösungsmittels oder in Substanz erfolgen. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie n-Hexan, Benzol, Toluol und Xylol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie Diethylether, Diphenylether Methyl-tert-butylether, Isopropylethylether, Dioxan, Dimethylglycol oder THF; Nitrilen wie Methylnitril, Butylnitril oder Phenylnitril; und Alkoholen wie Ethanol oder Isopropanol. Bevorzugte Lösungsmittel sind Toluol oder Mischungen aus Toluol und Ethanol.

Amine der Formel (IIIa) oder (IIIb) sind kommerziell erhältlich oder können nach literaturbekannten Verfahren hergestellt werden (vgl. 2-Fluor-ethylamin: US-Pat. 4030994 (1977); 3-Fluor-*n-*propylamin: US 6252087 B1; 3,3-Difluor-prop-2-enylamin Hydrochlorid: WO 2001/007414 A1; 3,3-Dichlor-prop-2-enylamin: DE 2747814; 2-Chlor-2-fluor-cyclopropylamin, 2,2-Dichlorcyclopropylamin: K. R. Gassen, B. Baasner, J. Fluorine Chem. 49, 127-139, 1990; Verbindungen der Formel (IIIa) oder (IIIb) in welcher R¹ für Alkyl steht, primäre Amine: vgl. z. B. Houben-Weyl, Methoden der Organischen Chemie, Bd. XI/1, 4. Aufl. 1957, Georg Thieme Verlag Stuttgart, S. 648; M. L. Moore in "The Leuckart Reaction" in: Organic Reactions, Bd. 5, 2. Aufl. 1952, New York, John Wiley & Sons, Inc. London).

Die Verbindungen der Formel (IVa) und (IVb) liegen als E/Z Gemisch vor.

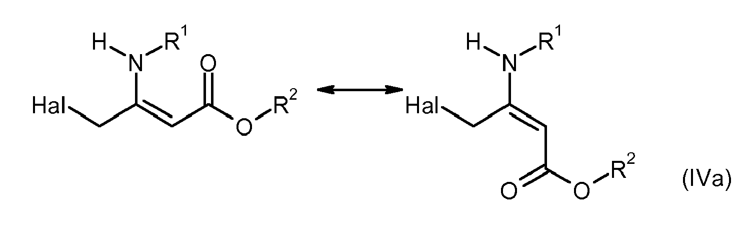

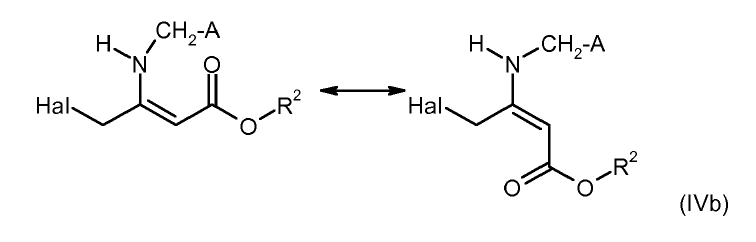

Zur Umsetzung der Verbindung der Formel (II) mit dem Amin der Formel (IIIa) oder (IIIb) kann ggf. eine Lewis-Säure als Katalysator zugesetzt werden. Beispiele hierfür sind Essigsäure, p-Toluolsulfonsäure, Trifluoressigsäure. Bevorzugt verwendet wird Essigsäure.

Das Amin mit der Formel (III) kann auch in Form des Salzes einsetzt werden. Dadurch kann der Zusatz an Lewis-Säure entfallen oder reduziert werden.

Die Umsetzung der Verbindung der Formel (II) mit dem Amin der Formel (IIIa) oder (IIIb) kann im Vakuum, bei Normaldruck oder unter Überdruck und bei Temperaturen von -20°C bis 200°C durchgeführt werden, vorzugsweise erfolgt die Reaktion bei Normaldruck und Temperaturen von - 20°C bis 60°C, besonders bevorzugt bei 10°C bis 40°C, am meisten bevorzugt bei 10°C bis 30°C. Ein Gegenstand der vorliegenden Erfindung ist daher auch ein Verfahren zur Herstellung von Verbindungen der Formel (IVa) oder (IVb) oder zur Herstellung von Verbindungen der Formel (I), wobei die Umsetzung der Verbindung der Formel (II) mit dem Amin der Formel (IIIa) oder dem Amin der Formel (IIIb) bei -20°C bis 60°C erfolgt. Die Reaktionsdauer beträgt zwischen 0,5 und 10 Stunden, längere Reaktionszeiten wirken sich nicht nachteilig aus. Das Lösungsmittel kann durch Abdestillieren oder im Vakuum im Temperaturbereich von 20°C bis 35°C entfernt werden.

Die Cyclisierung der Verbindung der Formel (IVa) oder (IVb) zur Verbindung der Formel (Va) oder (Vb) kann in einem inerten Lösungsmittel durchgeführt werden. Geeignete Lösungsmittel sind beispielsweise ausgewählt aus der Gruppe bestehend aus aliphatischen und aromatischen Kohlenwasserstoffen, wie n-Hexan, Benzol, Toluol und Xylol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie Diphenylether Methyl-tert-butylether, Isopropylethylether, Dioxan, Dimethylglycol oder THF; und Nitrilen wie Methylnitril, Butylnitril oder Phenylnitril; wobei Toluol bevorzugt ist.

Die Cyclisierung der Verbindung der Formel (IVa) oder (IVb) zur Verbindung der Formel (Va) oder (Vb) kann im Vakuum, bei Normaldruck oder unter Überdruck und bei Temperaturen von -20°C bis 200°C durchgeführt werden, bevorzugt bei 40°C - 150°C. Ein Gegenstand der vorliegenden Erfindung ist daher auch ein Verfahren zur Herstellung von Verbindungen der Formel (I), wobei die thermische Cyclisierung der Verbindung der Formel (IVa) zur Verbindung der Formel (Va) und der Verbindung der Formel (IVb) zur Verbindung der Formel (Vb) bei 40°C bis 150°C erfolgt. Die Reaktionsdauer beträgt zwischen 1 h und 10 Stunden, längere Reaktionszeiten wirken sich nicht nachteilig aus.

Die Isolierung der Verbindung der Formel (Va) oder (Vb) erfolgt durch Kristallisation oder durch Entfernen des Lösungsmittels.

Der Herstellung der 4-Aminobut-2-enolide der Formel (I) erfolgt durch Umsetzung der Verbindung der Formel (Va) mit Verbindungen der Formel (VIa) oder durch Umsetzung der Verbindung der Formel (Vb) mit Verbindungen der Formel (VIb).

Die Verbindungen der Formel (VIa) sind z. T. kommerziell erhältlich, teilweise bekannt und können nach bekannten Methoden erhalten werden (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 6-Methyl-3-chlormethyl-pyridin: EP 302389 A2, E. v der Eycken et al., J. Chem. Soc., Perkin Trans 2 5, 928-937 (2002); 6-Trifluormethyl-3-chlormethyl-pyridin: WO 2004/082616 A2; 2-Chlor-5-chlormethyl-pyrazin: JP 05239034 A2).

Allgemeine Wege zur Herstellung von Verbindungen der Formel (VIa) sind im folgenden Schema 4 wiedergegeben. 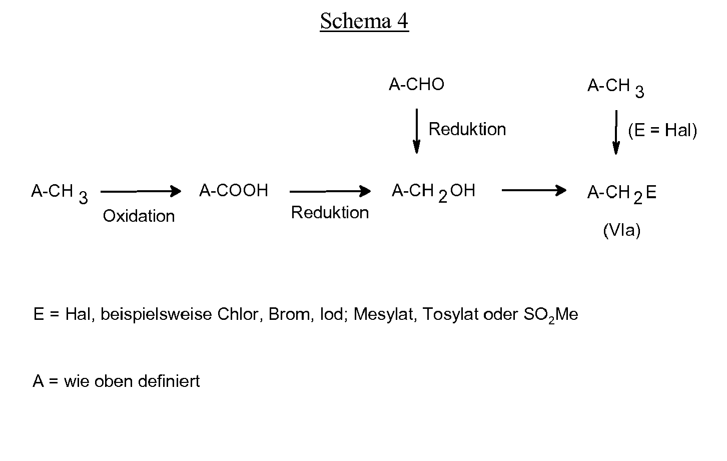 Beispielsweise können die heterocyclischen Carbonsäuren (A-COOH) nach literaturbekannten Methoden in die entsprechenden heterocyclischen Hydroxymethylverbindungen (A-CH₂-OH) überführt werden, die anschließend nach literaturbekannten Methoden zu aktivierten heterocyclischen Hydroxymethylverbindungen (A-CH₂-E, E = Tosylat, Mesylat) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Die Verbindungen der Formel (VIb) können z. T. kommerziell erhalten werden (vgl. z. B. Chlordifluormethan, 1-Brom-2-fluoretan, 2-Brom-1,1-difluorethan, 2-Brom-1-chlor-1-fluorethan, 1-Brom-3-fluorpropan, 3-Brom-1,1-difluor-prop-1-en) oder nach literaturbekannten Methoden erhalten werden (vgl. z. B. 3-Brom-1,1-dichlor-prop-1-en: WO 8800183 A1 (1988); Verbindungen der allgemeinen Formel (VIb) in welcher E für Halogen wie Chlor, Brom und Iod steht: Houben-Weyl, Methoden der Organischen Chemie, Bd, V/3, Georg Thieme Verlag Stuttgart, S. 503 und Bd. V/4 S. 13, 517; E¹ für Mesylat steht: Crossland, R. K., Servis, K. L. J. Org. Chem. (1970), 35, 3195; E für Tosylat steht: Roos, A. T. et al., Org. Synth., Coll. Vol. I, (1941), 145; Marvel, C. S., Sekera, V. C. Org. Synth., Coll. Vol. III, (1955), 366.

Als Abgangsgruppe E sind Gruppen geeignet, die bei den vorherrschenden Reaktionsbedingungen eine ausreichende Nucleofugizität aufweisen. Beispielhaft seien Halogene, wie Chlor, Brom, oder Jod, oder Mesylat, Tosylat oder SO₂Me als geeignete Abgangsgruppen genannt. Bevorzugt sind Chlor, Brom und Mesylat.

Die Reaktion erfolgt vorzugsweise in Gegenwart einer Base. Geeignete Basen sind organische und anorganische Basen, die üblicherweise in solchen Reaktionen verwendet werden. Vorzugsweise werden Basen verwendet, die beispielhaft ausgewählt sind aus der Gruppe bestehend aus Hydriden, Hydroxiden, Amiden, Alkoholaten, Acetaten, Fluoriden, Phosphaten, Carbonaten und Hydrogencarbonaten von Alkali- oder Erdalkalimetallen. Besonders bevorzugt sind dabei Natriumamid, Natriumhydrid, Lithiumdiisopropylamid, Natriummethanolat, Kalium-tert-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Ganz besonders bevorzugt sind Natriumhydroxid und Kaliumhydroxid. Ein Gegenstand der vorliegenden Erfindung ist daher auch ein Verfahren zur Herstellung von Verbindungen der Formel (I), wobei die Umsetzung der Verbindung der Formel (Va) mit der Verbindung der Formel (VIa) zur Verbindung der Formel (I) und die Umsetzung der Verbindung der Formel (Vb) mit der Verbindung der Formel (VIb) zur Verbindung der Formel (I) in Gegenwart von Natriumhydroxid oder Kaliumhydroxid erfolgt. Außerdem sind tertiäre Amine, wie Trimethylamin, Triethylamin, Tributylamin, N,N-Dimethylanilin, N,N-Dimethylbenzylamin, Pyridin, Alkylpyridine, wie 2-Methyl-5-Ethylpyridin, N-Methylpiperidin, N-Methylpyrolidon, N,N-Dimethylaminopyridin, Diazabicyclooctan (DABCO), Diazabicyclononen (DBN) und Diazabicycloundecen (DBU) bevorzugt.

Das molare Verhältnis von Base zu eingesetzten Tetronamid der Formel (Va) oder (Vb) beträgt beispielsweise 0,5 - 10, bevorzugt 0,9 - 6, besonders bevorzugt 1,0 - 2. Der Einsatz größerer Mengen an Base ist grundsätzlich möglich, führt jedoch zu keiner bevorzugten Ausführungsform und ist aus wirtschaftlichen Gründen nachteilig.

Das molare Verhältnis von Alkylierungsmittel der Formel (VIa) oder (VIb) zu eingesetztem Tetronamid der Formel (Va) oder (Vb) beträgt beispielsweise 0,5 - 3, bevorzugt 0,9 - 2, besonders bevorzugt 1,0 - 1,5. Der Einsatz größerer Mengen an Alkylierungsmittel ist grundsätzlich möglich, führt jedoch zu keiner bevorzugten Ausführungsform und ist aus wirtschaftlichen Gründen nachteilig.

Gegebenenfalls kann bei der Reaktion des Tetronamids der Formel (Va) oder (Vb) mit dem Alkylierungsmittel der Formel (VIa) oder (VIb) ein Phasentransferkatalysator eingesetzt werden, wie quartäre Ammonium- oder Phosphoniumverbindungen.

Die Reaktion des Tetronamides der Formel (Va) oder (Vb) mit dem Alkylierungsmittel der Formel (VIa) oder (VIb) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlorkohlenstoff, Dichlorethan oder Trichlorethan; Ether, wie Diethylether, Diisopropylether, Methyl-tert-butylether (MTBE), Methyl-tert-amylether, Dioxan, Tetrahydrofuran, Methyl-THF, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid (DMF), N,N-Dimethylacetamid, N-Methylformanilid, N-Methylpyrrolidon (NMP); oder Mischungen dieser.

Die Reaktion des Tetronamides der Formel (Va) oder (Vb) mit dem Alkylierungsmittel der Formel (VIa) oder (VIb) kann im Vakuum, bei Normaldruck oder unter Überdruck und bei Temperaturen von 0°C bis 150°C durchgeführt werden, vorzugsweise erfolgt die Reaktion bei Normaldruck und Temperaturen von 10 bis 90°C. Die Reaktionsdauer beträgt zwischen 1 h und 30 Stunden, längere Reaktionszeiten wirken sich nicht nachteilig aus.

Die abschließende Reinigung der 4-Aminobut-2-enoliden der Formel (I) kann durch übliche Reinigungsverfahren erfolgen. Vorzugsweise erfolgt die Reinigung durch Kristallisation.

Ein weiterer Gegenstand der vorliegenden Erfindung sind die Verbindungen der Formeln (IVa) und (IVb), wobei Hal für C1 steht.

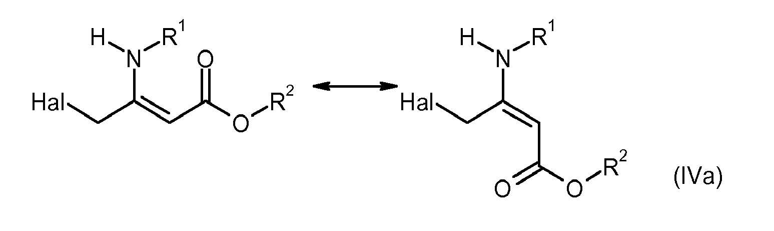

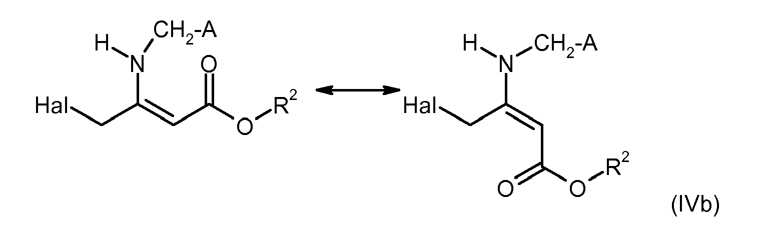

Die erfindungsgemäßen Verfahren zur Herstellung von 4-Aminobut-2-enoliden der Formel (I) und von Ausgangsprodukten zu deren Herstellung wird in den nachstehenden Beispielen beschrieben, welche die obige Beschreibung weiter illustrieren. Die Beispiele sind jedoch nicht in einschränkender Weise zu interpretieren.

### Herstellungsbeispiele:

### Beispiel 1

Zu einer Lösung von 300 g 4-Chloracetessigsäureethylester in 519 ml Toluol und 200 ml Ethanol werden 20,8 ml Essigsäure zugesetzt. Bei 10°C - 30°C werden unter Kühlung 188,7 g einer 33%igen Methylamin-Lösung in Ethanol zugetropft. Anschließend wird 8 h bei Raumtemperatur gerührt und das Lösungsmittel im Vakuum bei Temperaturen von bis zu 35°C entfernt. Man erhält 328 g 4-Chlor-3-(methylamino)but-2-encarbonsäureethylester in einer Reinheit von 91 % (dies entspricht 92 % Ausbeute).

¹H-NMR (CDCl₃, 298K) δ: 1,25 t (3H), 3,01 d (3H), 4,00 s (1H), 4,10 q (2H), 4,67 s + 4,96 s (1H) E/Z, 8,22 s (1H; NH)

### Beispiel 2

Zu einer Lösung von 30 g 4-Chloracetessigsäureethylester in 90 ml Toluol und 30 ml Ethanol werden 2 ml Essigsäure zugesetzt. Bei 10°C - 30°C werden unter Kühlung 8,9 g Ethylamin zugetropft. Anschließend wird 6 h bei Raumtemperatur gerührt und das Lösungsmittel im Vakuum bei Temperaturen von bis zu 35°C entfernt. Man erhält 37,7 g 4-Chlor-3-(ethylamino)but-2-encarbonsäureethylester in einer Reinheit von 88 % (dies entspricht 98 % Ausbeute).

¹H-NMR (DMSO d₆, 298K) δ: 1,16 m (6H), 2,81 q + 2,96 q (2H) E/Z, 3,32 q (2H), 4,32 s (2H), 4,43 s + 4,70 s (1H) E/Z 8,3 - 9,3 breit (1H,NH)

### Beispiel 3

Zu einer Lösung von 100 g 4-Chloracetessigsäureethylester in 300 ml Toluol und 100 ml Ethanol werden 7 ml Essigsäure zugesetzt. Bei 10°C - 30°C werden unter Kühlung 37,3 g n-Propylamin zugetropft. Anschließend wird 9 h bei Raumtemperatur gerührt und das Lösungsmittel im Vakuum bei Temperaturen von bis zu 35°C entfernt. Man erhält 140,6 g 4-Chlor-3-(n-propylamino)but-2-encarbonsäureethylester in einer Reinheit von 76 % (dies entspricht 88 % Ausbeute).

¹H-NMR (DMSO d₆, 298K) δ: 0,92 t (3H), 1,16 t (3H), 1,56 m (2H), 3,26 q (2H), 4,00 m (2H), 4,31 s (2H), 4,44 s + 4,72 s (1H) E/Z, 7,7 - 8,8 breit (1H,NH)

### Beispiel 4

Zu einer Lösung von 100 g 4-Chloracetessigsäureethylester in 300 ml Toluol und 100 ml Ethanol werden 7 ml Essigsäure zugesetzt. Bei 10°C - 30°C werden unter Kühlung 38,3 g iso-Propylamin zugetropft. Anschließend wird 5 h bei Raumtemperatur gerührt und das Lösungsmittel im Vakuum bei Temperaturen von bis zu 35°C entfernt. Man erhält 141,8 g 4-Chlor-3-(iso-propylamino)but-2-encarbonsäureethylester in einer Reinheit von 70 % (dies entspricht 82 % Ausbeute).

¹H-NMR (DMSO d₆, 298K) δ: 1,16 t (3H), 1,21 d (6H), 3,26 q (2H), 4,00 m (1H), 4,34 s (2H), 4,44 s + 4,69 s (1H) E/Z, 7,7 - 9,0 breit (1H, NH)

### Beispiel 5

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 48,3 g n-Butylamin zugetropft. Anschließend wird 8 h bei Raumtemperatur gerührt und das Lösungsmittel im Vakuum bei Temperaturen von bis zu 35°C entfernt. Man erhält 136,6 g 4-Chlor-3-(n-butylamino)but-2-encarbonsäureethylester in einer Reinheit von 89 % (dies entspricht 94 % Ausbeute).

¹H-NMR (DMSO d₆, 298K) δ: 0,92 t (3H), 1,16 t (3H), 1,35 m (2H), 1,52 m (2H), 3,29 q (2H), 4,01 m (2H), 4,44 s + 4,72 s (1H) E/Z, 7,7-8,5 breit (1H,NH)

### Beispiel 6

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 47,4 g iso-Butylamin zugetropft. Anschließend wird 8 h bei Raumtemperatur gerührt und das Lösungsmittel im Vakuum bei Temperaturen von bis zu 35°C entfernt. Man erhält 140,8 g 4-Chlor-3-(iso-butylamino)but-2-encarbonsäureethylester in einer Reinheit von 79 % (dies entspricht 86 % Ausbeute).

¹H-NMR (DMSO d₆, 298K) δ: 0,91 t (3H), 0,93 t (3H), 1,17 t (3H), 1,78 m (1H), 3,12 t (2H), 4,02 q (2H), 4,31 s (2H), 4,44 s + 4,69 s (1H) E/Z , 8,0 - 8,5 breit (1H,NH)

### Beispiel 7

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 46,5 g 2-Methoxyethylamin getropft. Anschließend wird 8 h bei Raumtemperatur gerührt und das Lösungsmittel im Vakuum bei Temperaturen von bis zu 35°C entfernt. Man erhält 140,8 g 4-Chlor-3-(2-methoxyethylamino)but-2-encarbonsäureethylester in einer Reinheit von 88 % (dies entspricht 95 % Ausbeute).

¹H-NMR (DMSO d₆, 298K) δ: 1,16 t (3H), 3,27 d (2H), 3,37 s (3H), 3,46 d (2H), 3,99 m (2H), 4,32 s (2H), 4,49 s + 4,72 s (1H) E/Z, 7,7-8,5 breit (1H,NH)

### Beispiel 8

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 65,7 g Benzylamin zugetropft. Anschließend wird 8 h bei Raumtemperatur gerührt und das Lösungsmittel im Vakuum bei Temperaturen von bis zu 35°C entfernt. Man erhält 157,8 g 4-Chlor-3-(benzylamino)but-2-encarbonsäureethylester in einer Reinheit von 50 % (dies entspricht 52 % Ausbeute).

¹H-NMR (DMSO d₆, 298K) δ: 1,11 t (3H), 3,95 q (2H), 4,24 d (2H), 4,34 s (2H), 4,44 s + 4,72 s ( 1H) E/Z , 7,20 - 7,40 m (5H), 8,3 - 8,8 breit (1H,NH)

### Beispiel 9

Zu einer Lösung von 2,1 g 4-Chloracetessigsäureethylester in 12 ml Toluol werden 0,15 ml Essigsäure zugesetzt. Bei 10°C - 30°C werden unter Kühlung 2 g 2-Chlor-5-(aminomethyl)pyridin gelöst in 4 ml Ethanol zugetropft. Anschließend wird 8 h bei Raumtemperatur gerührt und das Lösungsmittel im Vakuum bei Temperaturen von bis zu 35°C entfernt. Man erhält 4,2 g 4-Chlor-3-([[6-chlorpyridin-3-yl]methyl]amino)but-2-encarbonsäureethylester in einer Reinheit von 86 % (dies entspricht 98 % Ausbeute).

¹H-NMR (CDCl₃, 298K) δ: 1,26 t (3H), 3,98 s (2H), 4,13 q (2H), 4,56 d (2H), 4,,79 s + 4,99 s (1H) E/Z, 7,35 d (1H), 7,64 d (1H), 8,35 d (1H), 8,5 - 8,7 breit (1H,NH)

### Beispiel 10

Zu einer Lösung von 197,2 g 4-Chloracetessigsäureethylester in 1080 ml Toluol werden 13,3 ml Essigsäure zugesetzt. Bei 10°C - 30°C werden unter Kühlung 100 g 2,2-Difluorethylamin gelöst in 360 ml Ethanol zugetropft. Anschließend wird 8 h bei Raumtemperatur gerührt und das Lösungsmittel im Vakuum bei Temperaturen von bis zu 35°C entfernt. Man erhält 278 g 4-Chlor-3-(2,2-difluorethylamino)but-2-encarbonsäureethylester in einer Reinheit von 93% (dies entspricht 98% Ausbeute).

¹H-NMR (DMSO d₆, 298K) δ: 1,17 t (3H), 3,73 m (2H), 4,04 q (2H), 4,36 s (2H), 4,84 s (1H), 6,03 t + 6,16 t + 6,31 t (1 H, CHF₂), 8,2 - 8,4 t (breit) (1H,NH)

### Beispiel 11

328,8 g 4-Chlor-3-(methylamino)but-2-encarbonsäureethylester (Beispiel 1) werden in 519 g Toluol suspendiert und 4 h unter Rückfluss erhitzt. Anschließend wird auf 20°C abgekühlt, der Feststoff abfiltriert und mit 150 ml Toluol und mit 150 ml Ethanol gewaschen. Man erhält 142 g 4-(Methylamino)furan-2(5H)-on mit einer Reinheit von 95 % (dies entspricht 71% Ausbeute).

¹H-NMR (DMSO d₆, 298K) δ: 2,71 d (3H), 4,50 s (1H), 4,60 s (2H), 7,2 - 7,7 breit (1H,NH)

### Beispiel 12

37,5 g 4-Chlor-3-(ethylamino)but-2-encarbonsäureethylester (Beispiel 2) werden in 86,5 g Toluol suspendiert und 2 h unter Rückfluss erhitzt. Anschließend wird auf 20°C abgekühlt und das Lösungsmittel im Vakuum entfernt. Man erhält 25,5 g 4-(Ethylamino)furan-2(5H)-on mit einer Reinheit von 74 % (dies entspricht 75% Ausbeute).

¹H-NMR (DMSO d₆, 298K) δ: 1,12 t (3H), 3,07 m (2H), 4,51 s (1H), 4,60 s (2H), 7,4 - 7,7 breit (1H,NH)

### Beispiel 13

140 g 4-Chlor-3-(n-propylamino)but-2-encarbonsäureethylester (Beispiel 3) werden in 216 g Toluol gelöst und 2 h unter Rückfluss erhitzt. Anschließend wird auf 20°C abgekühlt und das Lösungsmittel im Vakuum entfernt. Man erhält 100,3 g 4-(n-Propylamino)furan-2(5H)-on mit einer Reinheit von 86 % (dies entspricht 89% Ausbeute).

¹H-NMR (DMSO d₆, 298K) δ: 0,89 t (3H), 1,51 m (2H), 3,00 q (2H), 4,52 s (1H), 4,60 s (1H), 7,4 -7,6 breit (1H,NH)

### Beispiel 14

50 g 4-Chlor-3-(n-butylamino)but-2-encarbonsäureethylester (Beispiel 5) werden in 86 g Toluol gelöst und 2 h unter Rückfluss erhitzt. Anschließend wird auf 20°C abgekühlt und das Lösungsmittel im Vakuum entfernt. Man erhält 40,7 g 4-(n-Butylamino)furan-2(5H)-on mit einer Reinheit von 86 % (dies entspricht 99 % Ausbeute).

¹H-NMR (DMSO d₆, 298K) δ: 0,89 t (3H), 1,33 m (2H), 1,50 m (2H), 3,01 q (2H), 4,51 s (1H), 4,59 s (2H), 7,4 -7,7 breit (1H, NH)

### Beispiel 15

140 g 4-Chlor-3-(iso-Butylamino)but-2-encarbonsäureethylester (Beispiel 6) werden in 216 g Toluol gelöst und 2 h unter Rückfluss erhitzt. Anschließend wird auf 20°C abgekühlt und das Lösungsmittel im Vakuum entfernt. Man erhält 104 g 4-(iso-Butylamino)furan-2(5H)-on mit einer Reinheit von 87 % (dies entspricht 92% Ausbeute).

¹H-NMR (DMSO d₆, 298K) δ: 0,89 d (6H), 1,79 m (1H), 2,84 t (2H), 4,52 s (1H), 4,61 s (2H), 7,4 - 7,7 breit (1H, NH)

### Beispiel 16

157 g 4-Chlor-3-(Benzylamino)but-2-encarbonsäureethylester (Beispiel 8) werden in 259 g Toluol gelöst und 4 h unter Rückfluss erhitzt. Anschließend wird auf 20°C abgekühlt, der Feststoff abfiltriert und mit 100 ml Toluol gewaschen. Man erhält 109 g 4-(Benzylamino)furan-2(5H)-on mit einer Reinheit von 92 % (dies entspricht 86% Ausbeute).

¹H-NMR (DMSO d₆, 298K) δ: 4,27 d (2H), 4,58 s (1H), 4,67 s (2H), 7,26 - 7,45 m (5H), 7,9 - 8,2 breit (1 H,NH)

### Beispiel 17

18,8 g 4-Chlor-3-([[6-chlorpyridin-3-yl]methyl]amino)but-2-encarbonsäureethylester (Beispiel 9) werden in 86 g Toluol gelöst und 9 h unter Rückfluss erhitzt. Anschließend wird auf 20°C abgekühlt und das Lösungsmittel im Vakuum entfernt. Man erhält 16,1 g 4-([[6-chlorpyridin-3-yl]methyl]amino)furan-2(5H)-on mit einer Reinheit von 96 % (dies entspricht 87% Ausbeute).

¹H-NMR (CDCl₃, 298K) δ: 4,33 d (2H), 4,66 s (1H), 4,72 s (2H), 6,5 - 6,7 breit (1H,NH), 7,32 d (1H), 7,63 d (1H), 8,34 d (1H)

### Beispiel 18

280 g 4-Chlor-3-(2,2-Difluorethylamino)but-2-encarbonsäureethylester (Beispiel 10) werden in 709 g Toluol gelöst und 4 h unter Rückfluss erhitzt. Anschließend wird auf 20°C abgekühlt, der Feststoff abfiltriert und mit 100 ml Toluol gewaschen. Man erhält 183 g 4-(Difluorethylamino)furan-2(5H)-on mit einer Reinheit von 97 % (dies entspricht 96% Ausbeute).

¹H-NMR (DMSO d₆, 298K) δ: 3,44 - 3,59 m (2H), 4,65 s (1H), 4,77 s (2H), 6,60 t + 6,14 t + 6,28 t (1 H, CHF₂), 7,4 - 7,9 breit (1H,NH)

### Beispiel 19

30 g 4-(Methylamino)furan-2(5H)-on (Beispiel 11) werden in 450 ml Dimethoxyethan bei Raumtemperatur vorgelegt. Anschließend werden 12,6 g Natriumhydroxid zugesetzt und bei 40 °C werden 225 g einer 20%igen Lösung von 2-Chlor-5-(chlormethyl)pyridin in Dimethoxyethan zudosiert. Es wird noch weitere 6 h bei 50°C gerührt. Das Lösungsmittel wird weitgehend im Vakuum entfernt und der Rückstand mit 300 ml Wasser versetzt. Der Feststoff wird abfiltriert, mit 150 ml Wasser gewaschen und im Vakuum getrocknet. Man erhält 58,7 g 4-([[6-chlorpyridin-3-yl]methyl](methyl)amino)furan-2(5H)-on mit einer Reinheit von 94 % (dies entspricht 87% Ausbeute).

¹H-NMR (DMSO d₆, 298K) δ: 2,88 s (3H), 4,47 s (2H), 4,47 s (1H), 4,89 s (2H), 7,52 d (1H), 7,78 d (1H), 8,37 s (1H)

## Patentansprüche

1. Verfahren zum Herstellen von Verbindungen der Formel (IVa) oder (IVb) 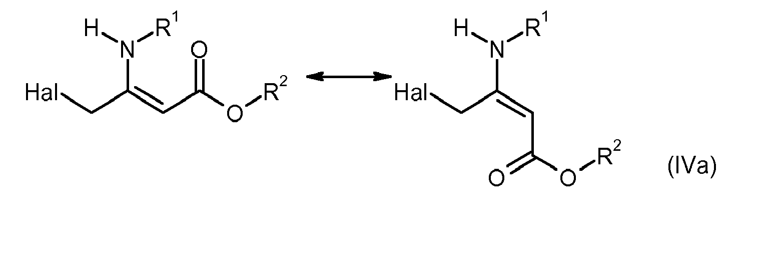 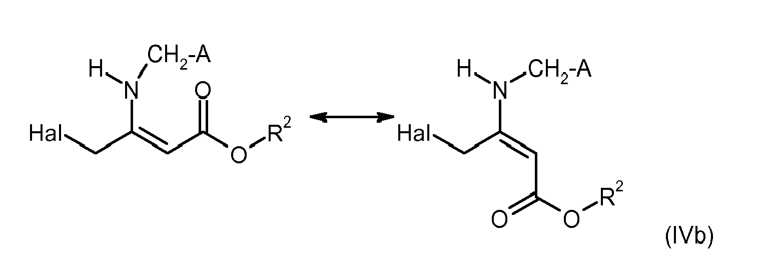 in welcher
A für Pyrid-2-yl oder Pyrid-4-yl steht oder für Pyrid-3-yl, welches gegebenenfalls in 6- Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor- pyrazin-5-yl oder für 1,3-Thiazol-5-yl, welches gegebenenfalls in 2-Position substituiert ist durch Chlor oder Methyl, oder
A für einen Rest Pyrimidinyl, Pyrazolyl, Thiophenyl, Oxazolyl, Isoxazolyl, 1,2,4-Oxa- diazolyl, Isothiazolyl, 1,2,4-Triazolyl oder 1,2,5-Thiadiazolyl steht, welcher gegebe- nenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃- Alkylsulfonyl (welches gegebenenfalls durch Fluor und/oder Chlor substituiert ist), substituiert ist,
oder
A für einen Rest 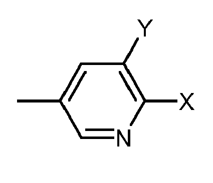 steht,
in welchem
X für Halogen, Alkyl oder Halogenalkyl steht
Y für Halogen, Alkyl, Halogenalkyl, Halogenalkoxy, Azido oder Cyan steht,
R¹ für Alkyl, Halogenalkyl, Alkenyl, Halogenalkenyl, Alkinyl, Cycloalkyl, Cycloalkylalkyl, Halogencycloalkyl, Alkoxy, Alkoxyalkyl, Halogencycloalkylalkyl oder Arylalkyl steht,
R² für C₁-C₁₂-Alkyl, C₅-C₈-Aryl oder Arylalkyl, bevorzugt für C₁-C₆ Alkyl, besonders bevorzugt für Methyl oder Ethyl steht und
Hal für Cl, Br, oder I steht, bevorzugt für Cl oder Br, besonders bevorzugt für Cl,
**dadurch gekennzeichnet, dass**
4-Halogenacetessigester der allgemeinen Formel (II) 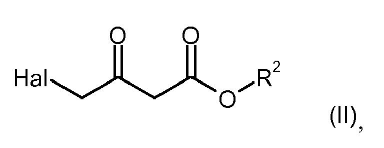 in welcher R² und Hal die oben genannte Bedeutung haben,
a) mit einem Amin der Formel (IIIa) 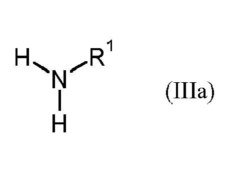 in welcher R¹ die oben genannte Bedeutung hat,
zur Verbindung der Formel (IVa) umgesetzt wird,
oder
b) mit einem Amin der Formel (IIIb)
H₂N-CH₂-A (IIIb)
in welcher A die oben genannte Bedeutung hat,
zur Verbindung der Formel (IVb) umgesetzt wird.

2. Verfahren zum Herstellen von 4-Aminobut-2-enoliden der Formel (I) 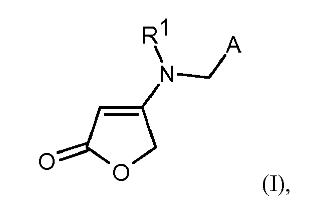 in welcher A und R¹ wie in Anspruch 1 definiert sind,
**dadurch gekennzeichnet, dass**
4-Halogenacetessigester der allgemeinen Formel (II) 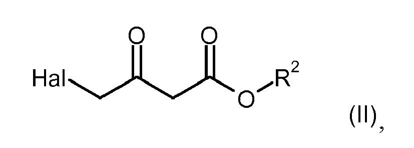 in welcher
R² für C₁-C₁₂-Alkyl, C₅-C₈-Aryl oder Arylalkyl, bevorzugt für C₁-C₆ Alkyl, besonders bevorzugt für Methyl oder Ethyl steht, und
Hal für Cl, Br oder I steht, bevorzugt für Cl oder Br, besonders bevorzugt für Cl,
a) mit einem Amin der Formel (IIIa) 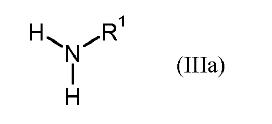 in welcher R¹ die oben genannte Bedeutung hat,
zu einer Verbindung der Formel (IVa) 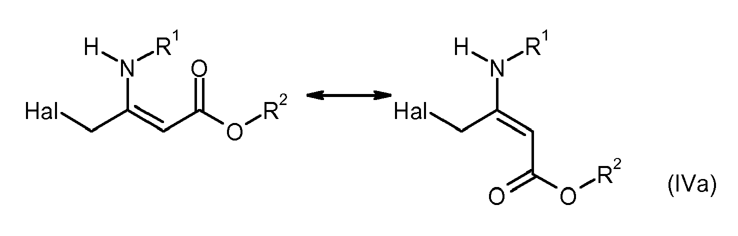 umgesetzt wird, anschließend die Verbindung der Formel (IVa) in Gegenwart eines Lösungsmittels thermisch zu einer Verbindung der Formel (Va) cyclisiert wird 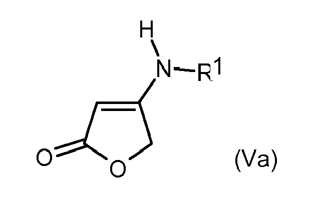 und die Verbindung der Formel (Va) im letzten Schritt mit einer Verbindung der Formel (VIa)
A-CH₂-E (VIa)
wobei A die oben genannte Bedeutung hat und E für eine Abgangsgruppe steht, zu Verbindung (I) umgesetzt wird; oder
b) mit einem Amin der Formel (IIIb)
H₂N-CH₂-A (IIIb)
in welcher A die oben genannte Bedeutung hat,
zu einer Verbindung der Formel (IVb) 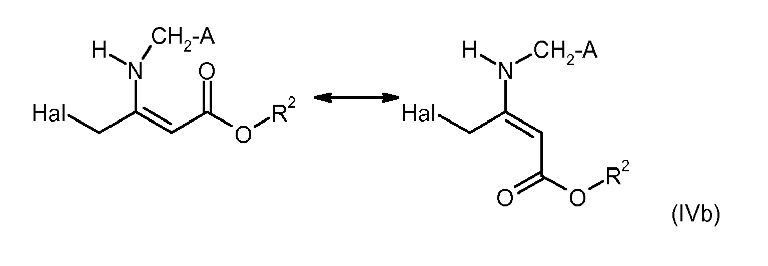 umgesetzt wird, anschließend die Verbindung der Formel (IVb) in Gegenwart eines Lösungsmittels thermisch zu einer Verbindung der Formel (Vb) cyclisiert wird 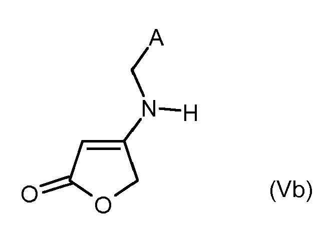 und die Verbindung der Formel (Vb) im letzten Schritt mit einer Verbindung der Formel (VIb)
R¹-E (VIb)
wobei R¹ die oben genannte Bedeutung hat und E für eine Abgangsgruppe steht, zu Verbindung (I) umgesetzt wird.

3. Verfahren nach Anspruch 1 oder 2, **dadurch gekennzeichnet, dass** die Umsetzung der Verbindung der Formel (II) mit dem Amin der Formel (IIIa) oder dem Amin der Formel (IIIb) bei -20°C bis 60°C erfolgt.

4. Verfahren nach Anspruch 2 oder 3, **dadurch gekennzeichnet, dass** die thermische Cyclisierung der Verbindung der Formel (IVa) zur Verbindung der Formel (Va) und der Verbindung der Formel (IVb) zur Verbindung der Formel (Vb) bei 40°C bis 150°C erfolgt.

5. Verbindung der Formel (IVa) 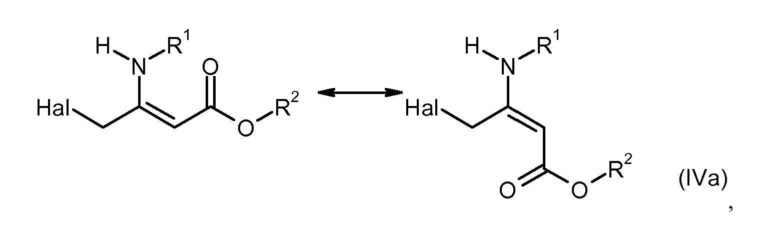 in welcher
Hal für Chlor steht,
R¹ für C₁₋₁₂-Alkyl, Halogenalkyl, Alkenyl, Halogenalkenyl, Alkinyl, Cycloalkyl, Cycloalkylalkyl, Halogencycloalkyl, Alkoxy oder Halogencycloalkylalkyl steht und
R² für C₁-C₁₂-Alkyl, C₅-C₈-Aryl oder Arylalkyl, bevorzugt für C₁-C₆ Alkyl, besonders bevorzugt für Methyl oder Ethyl steht.

6. Verbindung der Formel (IVb) 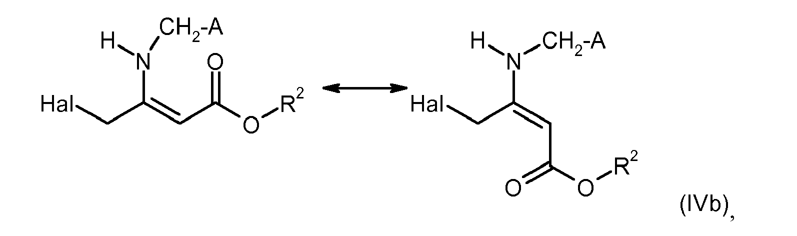 in welcher
Hal für Chlor steht.
R² für C₁-C₁₂-Alkyl, C₅-C₈-Aryl oder Arylalkyl steht, bevorzugt für C₁-Cy Alkyl, besonders bevorzugt für Methyl oder Ethyl und
A für Pyrid-2-yl oder Pyrid-4-yl steht oder für Pyrid-3-yl, welches gegebenenfalls in 6- Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor- pyrazin-5-yl oder für 1,3-Thiazol-5-yl, welches gegebenenfalls in 2-Position substituiert ist durch Chlor oder Methyl, oder
A für einen Rest Pyrimidinyl, Pyrazolyl, Thiophenyl, Oxazolyl, Isoxazolyl, 1,2,4-Oxa- diazolyl, Isothiazolyl, 1,2,4-Triazolyl oder 1,2,5-Thiadiazolyl steht, welcher gegebe- nenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃- Alkylsulfonyl (welches gegebenenfalls durch Fluor und/oder Chlor substituiert ist), substituiert ist,
oder
A für einen Rest 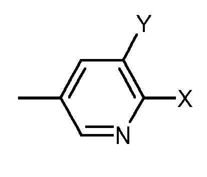 steht,
in welchem
X für Halogen, Alkyl oder Halogenalkyl steht und
Y für Halogen, Alkyl, Halogenalkyl, Halogenalkoxy, Azido oder Cyan steht.
